(19) Europäisches Patentamt
European Patent Office
Office européen des brevets

(11) Numéro de publication: **0 004 494**
**B1**

(12) **FASCICULE DE BREVET EUROPEEN**

(45) Date de publication du fascicule de brevet: **17.02.82**

(51) Int. Cl.³: **C 07 D 405/14,**
**A 61 K 31/445**

(21) Numéro de dépôt: **79400145.3**

(22) Date de dépôt: **06.03.79**

(54) **Nouveaux dérivés de 1,3-dihydro 3-(1-(2-(2,3-dihydro 1,4-benzodioxin-2-yl)2-hydroxy éthyl)pipéridin-4-yl)2H-indol 2-one, leur procédé de préparation, leur application comme médicaments et les compositions pharmaceutiques les renfermant.**

(30) Priorité: **22.03.78 FR 7808292**

(43) Date de publication de la demande:
**03.10.79 Bulletin 79/20**

(45) Mention de la délivrance du brevet:
**17.02.82 Bulletin 82/7**

(84) Etats Contractants Désignés:
**BE CH DE GB IT NL SE**

(56) Documents cités:
**FR - A - 2 213 059**
**FR - A - 2 349 331**

(73) Titulaire: **ROUSSEL-UCLAF**
**102, route de Noisy Boîte postale no.9**
**F-93230 Romainville (FR)**

(72) Inventeur: **Clemence, François**
**2, rue Turgot**
**F-75009 Paris (FR)**
Inventeur: **Humbert, Daniel**
**15, rue Gaston Charle**
**F-94120 Fontenay sous Bois (FR)**
Inventeur: **Fournex, Robert**
**8, rue du Commandant Rivière**
**F-75008 Paris (FR)**

(74) Mandataire: **Niel, Michel et al,**
**boîte postale no 9 102, route de Noisy**
**F-93230 Romainville (FR)**

**EP 0 004 494 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouveaux dérivés de 1,3-dihydro 3-/1-/2-(2,3-dihydro 1,4-benzodioxin-2-yl) 2-hydroxy éthyl/ pipéridin-4-yl/ 2H-indol 2-one, leur procédé de préparation, leur application comme médicaments et les compositions pharmaceutiques les renfermant

La présente invention concerne de nouveaux dérivés de 1,3-dihydro 3-/1-/2-(2,3-dihydro 1,4-benzodioxin-2-yl) 2-hydroxy éthyl/ pipéridin-4-yl/ 2H-indol-2-one, leur procédé de préparation, leur application comme médicaments et les compositions pharmaceutiques les renfermant.

La demande de brevet français FR—A—2 349 331 décrit des produits de formule:

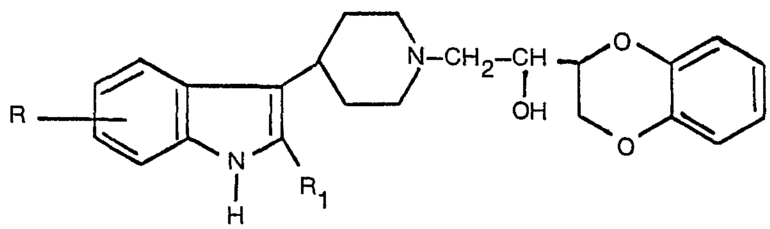

dans laquelle R représente un atome d'hydrogène ou un radical alcoxy contenant de 1 à 5 atomes de carbone, $R_1$ représente un atome d'hydrogène ou un radical alcoyle contenant de 1 à 5 atomes de carbone, ainsi que leurs sels d'addition avec les acides minéraux et organiques.

La demande de brevet français FR—A—2 213 059 décrit des produits de formule:

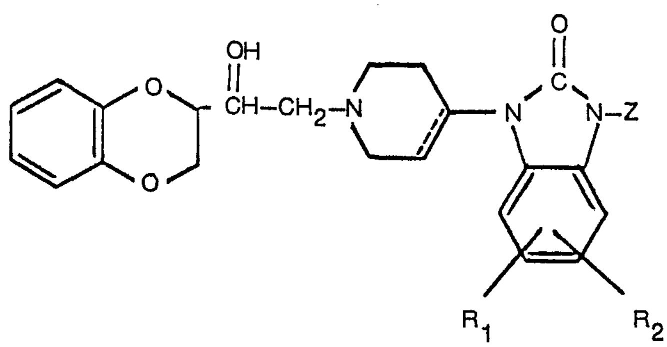

et leurs sels d'addition d'acides thérapeutiquement actifs et leurs formes isomères optiques stéréochimiques, dans laquelle formule: Z est choisi dans le groupe formé par l'hydrogène, les radicaux alcoyle inférieur, alcényle inférieur, (alcoyl inférieur) oxy-(alcoyle inférieur), phénoxy-(alcoyle inférieur), (alcoyl inférieur)-carbonyl-(alcoyle inférieur), cyano-(alcoyle inférieur), phényl-(alcoyl inférieur), hydroxy-(alcoyle inférieur), di-(alcoyle inférieur)-amino-(alcoyle inférieur) (alcoylène inférieur cyclique) amino-alcoyle inférieur, et (alcoyl inférieur) oxy-carbonyl-(alcoyle inférieur); et $R_2$ représentent chacun l'hydrogène, un halogène ou un alcoyle inférieur; et la ligne discontinue indique une double liaison éventuelle.

La présente invention a pour objet les produits de formule générale I:

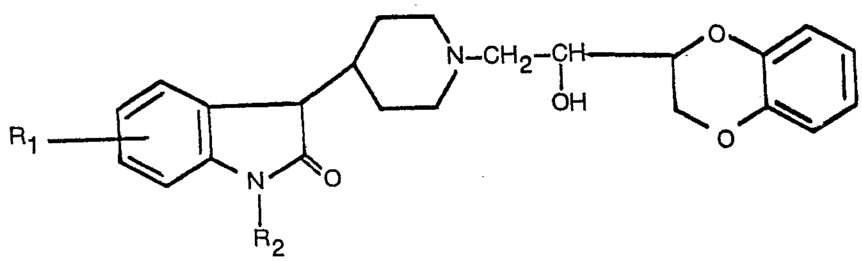

(I)

dans laquelle $R_1$ représente un atome d'hydrogène, un atome de chlore, de brome ou de fluor, un radical alcoxy contenant de 1 à 5 atomes de carbone, $R_2$ représente un atome d'hydrogène ou un radical alcoyle contenant de 1 à 5 atomes de carbone, sous leurs formes racémiques ou optiquement actives, ainsi que leurs sels d'addition avec les acides minéraux ou organiques.

Le terme radical alcoxy contenant de 1 à 5 atomes de carbone peut désigner, par exemple, un radical méthoxy, éthoxy, propoxy, butoxy, sec-butoxy ou tert-butoxy.

Le terme radical alcoyle contenant de 1 à 5 atomes de carbone peut désigner, par exemple, un radical méthyle, éthyle, propyle, butyle, sec-butyle, tert-butyle, pentyle.

Les sels d'addition avec les acides minéraux ou organiques peuvent être, par exemple, les sels

formés avec les acides chlorhydrique, bromhydrique, iodhydrique, nitrique, sulfurique, phosphorique, acétique, formique, benzoïque, maléique, fumarique, succinique, tartrique, citrique, oxalique, glyoxylique, aspartique, les acides alcoylmonosulfoniques, tels que l'acide méthanesulfonique, l'acide éthanesulfonique, l'acide propane sulfonique, les acides alcoyldisulfoniques tels que l'acide méthane-disulfonique, l'acide $\alpha,\beta$-éthanedisulfonique, les acides arylmonosulfoniques, tels que l'acide benzène sulfonique et les acides aryldisulfoniques.

Parmi les produits et sels, objet de l'invention, on retient plus particulièrement les produits tels que définis par la formule I ci-dessus, caractérisés en ce que dans ladite formule I, $R_1$ représente un atome d'hydrogène, un atome de chlore ou un radical méthoxy et $R_2$ représente un atome d'hydrogène ou un radical méthyle sous leurs formes racémiques ou optiquement actives, ainsi que leurs sels d'addition avec les acides minéraux ou organiques.

Parmi ces produits, on peut citer plus particulièrement:

— l'érythro 1,3-dihydro 3-/1-/2-(2,3-dihydro 1,4-benzodioxin-2-yl) 2-hydroxy éthyl/ pipéridin-4-yl/ 2H-indol 2-one et son chlorhydrate sous leurs formes racémiques et optiquement actives,

— le thréo 1,3-dihydro 3-/1-/2-(2,3-dihydro 1,4-benzodioxin-2-yl) 2-hydroxy éthyl/ pipéridin-4-yl/ 2H-indol 2-one et son chlorhydrate sous leurs formes racémiques et optiquement actives,

— l'érythro 1,3-dihydro 3-/1-/2-(2,3-dihydro 1,4-benzodioxin-2-yl) 2-hydroxyéthyl/ pipéridin-4-yl/ 1-méthyl 2H-indol 2-one et son chlorhydrate sous leurs formes racémiques et optiquement actives,

— l'érythro 1,3-dihydro 3-/1-/2-(2,3-dihydro 1,4-benzodioxin-2-yl) 2-hydroxy éthyl/ pipéridin-4-yl/ 5-methoxy 2H-indol 2-one et son chlorhydrate sous leurs formes racémmiques et optiquement actives.

L'invention a également pour objet un procédé de préparation des produits tels que définis par la formule I ci-dessus, sous leurs formes racémiques et optiquement actives, ainsi que de leurs sels d'addition avec les acides minéraux et organiques, caractérisé en ce que l'on fait réagir un produit de formule II:

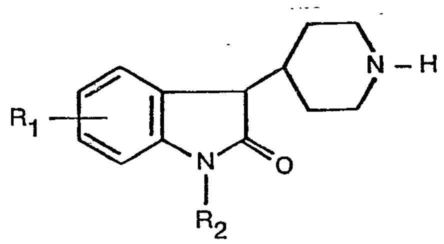

(II)

dans laquelle $R_1$ et $R_2$ ont la signification donnée ci-dessus, avec un produit de formule III:

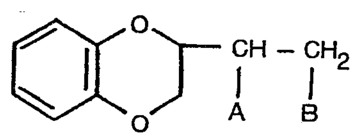

(III)

dans laquelle A et B représentent ensemble un atome d'oxygène ou bien A représente un radical hydroxy et B représente un atome de chlore ou de brome, pour obtenir le produit de formule I que, si désiré, on soumet à l'action d'un acide minéral ou organique pour en former le sel.

Dans des conditions préférentielles de mise en oeuvre de l'invention, le procédé ci-dessus décrit est réalisé de la manière suivante:

— lorsque le produit de formule III utilisé est un produit de formule III dans laquelle A et B représentent ensemble un atome d'oxygène, la réaction d'un tel produit avec le produit de formule II est réalisée de préférence au sein d'un solvant organique tel qu'un hydrocarbure aromatique comme le benzène, le toluène, le xylène, un alcanol comme le méthanol, l'éthanol, le propanol, un hydrocarbure halogéné comme le chlorure de méthylène, le chloroforme ou un analogue ou un mélange de ces solvants tel que par exemple un mélange d'un hydrocarbure aromatique et d'un alcanol inférieur,

— lorsque le produit de formule III utilisé est un produit de formule III dans laquelle A représente un radical hydroxy et B représente un atome d'halogène, la réaction est réalisée au sein d'un solvant organique tel que, par exemple, un hydrocarbure aromatique comme le benzène, le toluène, le xylène; un alcanol inférieur comme l'alcool éthylique, l'alcool butylique, l'alcool amylique; une cétone comme l'acétone, la méthyléthylcétone, la méthylisobutylcétone; un éther comme le dioxane; un amide comme le diméthylformamide. On utilise de préférence un excès de produit de formule III.

La réaction du produit de formule II avec le produit de formule III peut aussi être réalisée en présence d'un agent basique tel que, par exemple, un carbonate ou un bicarbonate alcalin.

La réaction est réalisée à une température allant de la température ambiante à la température de reflux du mélange réactionnel.

Les produits de formules II et III peuvent être utilisés, bien entendu, sous l'une quelconque de leur forme d'isomérie.

La réaction du produit de formule I avec un acide minéral ou organique est réalisée en présence d'un solvant ou d'un mélange de solvants tels que l'eau, l'éther éthylique ou l'acétone.

Les produits de formule I ci-dessus peuvent exister sous différentes formes d'isomérie optique stéréochimique et la présente invention a également pour objet ces différentes formes.

Elles peuvent être obtenues séparément par des méthodes connues en soi. Les racémates diastéréoisomères dénommés par les préfixes érythro et thréo peuvent être obtenus séparément par des méthodes connues, par exemple, par cristallisation sélective, par chromatographie sur colonne ou par la préparation dirigée dudit produit de formule I à partir de la formule adéquate du produit de formule III et/ou de la forme adéquate du produit de formule II.

Des exemples de préparations dirigées à partir de produits de formule III sont donnés ci-après dans la partie expérimentale.

Ces racémates érythro et thréo peuvent être résolus en leurs énantiomères optiques par des méthodes également connues, telles que, par exemple, la formation de sels au moyen d'acides optiquement actifs.

Bien entendu, les mélanges de différents isomères de produits de formule I et, en particulier, les mélanges de racémates diastéréoisomères desdits produits, entrent dans le cadre de l'invention.

Les produits de formule II, peuvent être préparés par réaction d'un produit de formule (A):

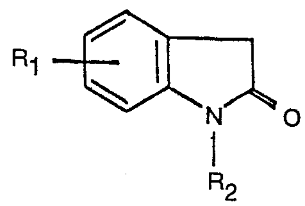

(A)

dans laquelle $R_1$ et $R_2$ ont la signification indiquée ci-dessus avec le produit de formule (B):

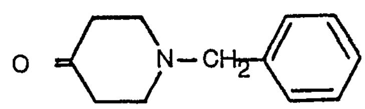

(B)

suivie du traitement du produit de formule IV obtenu:

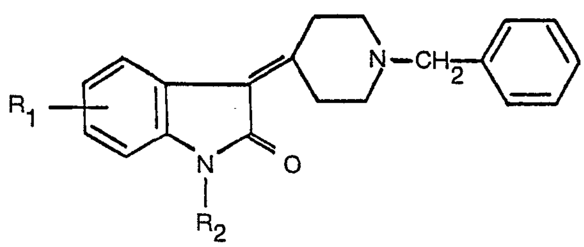

(IV)

par un agent de réduction et de débenzylation.

Dans des conditions préférentielles de mise en oeuvre de ce dernier procédé, on opère de la manière suivante:

— La réaction du produit de formule A avec le produit de formule B est réalisée en présence d'un agent basique tel que l'ammoniac, une amine comme la monoéthylamine, la diéthylamine, la triéthylamine, la pyrrolidine, la pipéridine.

— La réaction du produit de formule A avec le produit de formule B est réalisée au sein d'un solvant organique tel qu'un alcool comme le méthanol, l'éthanol et le propanol. La réaction est réalisée à une température comprise entre la température ambiante et la température de reflux du milieu réactionnel.

— L'agent de réduction et de débenzylation peut être l'hydrogène en présence d'un catalyseur tel que le palladium, le platine.

— La réaction de réduction et débenzylation est réalisée au sein d'un solvant organique tel qu'un alcool, comme le méthanol, l'éthanol, le propanol ou un acide, comme l'acide acétique.

Les produits de formule I tels que définis ci-dessus et leurs sels d'acides minéraux et organiques possèdent d'intéressantes propriétés pharmacologiques.

Ils abaissent notamment la pression sanguine; ils manifestent une importante activité antihypertensive.

Ces propriétés justifient leur application en thérapeutique et l'invention a également pour objet, à titre de médicaments, les produits tels que définis par la formule I ci-dessus sous leurs formes racémiques et optiquement actives, ainsi que leurs sels d'addition avec les acides minéraux et organiques pharmaceutiquement acceptables.

L'invention a plus particulièrement pour objet des médicaments tels que définis ci-dessus, caractérisés en ce que, dans ladite formule I, $R_1$ représente un atome d'hydrogène, un atome de chlore ou un radical méthoxy, et $R_2$ représente un atome d'hydrogène ou un radical méthyle, sous leurs formes racémiques ou optiquement actives, ainsi que leurs sels d'addition avec les acides minéraux ou organiques pharmaceutiquement acceptables.

L'invention a tout spécialement pour objet, à titre de médicaments:

— l'érythro 1,3-dihydro 3-/1-/2-(2,3-dihydro 1,4-benzodioxin-2-yl) 2-hydroxy éthyl/ pipéridin-4-yl/ 2H-indol 2-one et son chlorhydrate sous leurs formes racémiques et optiquement actives,

— le thréo 1,3-dihydro 3-/1-/2-(2,3-dihydro 1,4-benzodioxin-2-yl) 2-hydroxy éthyl/ pipéridin 4-yl/ 2H-indol 2-one et son chlorhydrate, sous leurs formes racémiques et optiquement actives,

— l'érythro 1,3-dihydro 3-/1-/2-(2,3-dihydro 1,4-benzodioxin-2-yl) 2-hydroxy éthyl/ pipéridin-4-yl/ 1-méthyl 2H-indol 2-one et son chlorhydrate sous leurs formes racémiques et optiquement actives,

— l'érythro 1,3-dihydro 3-/1-/2-(2,3-dihydro 1,4-benzodioxin-2-yl) 2-hydroxy éthyl/ pipéridin-4-yl/ 5-méthoxy 2H-indol 2-one et son chlorhydrate sous leurs formes racémiques et optiquement actives.

Ces produits et sels constituent des médicaments très utiles en thérapeutique humaine, notamment dans le traitement de l'hypertension artérielle sous toutes ses formes: permanente, légère, modérée ou sévère. La posologie, variable selon le produit ou sel utilisé, peut s'échelonner par exemple entre 0,100 et 2 g par jour chez l'adulte par voie orale.

La présente invention a ainsi pour objet les compositions pharmaceutiques contenant, à titre de principe actif, l'un au moins des médicaments précités. Ces compositions sont réalisées de façon à pouvoir être administrés par la voie digestive ou parentérale. Elles peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables; elles sont préparées selon les méthodes usuelles.

Le/ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végetale, les dérivés paraffiniques, les glycols, les divers agents mouillants, dispersants ou émulsifiants, les conservateurs.

Les exemples suivants illustrent l'invention sans toutefois la limiter.

## Exemple 1

*3-/1-/2-(2,3-dihydro 1,4-benzodioxin-2-yl) 2-hydroxy éthyl/pipéridin-4-yl) 1,3-dihydro 2H-indol 2-one et son chlorhydrate*

On porte au reflux sous azote pendant 2 heures un mélange composé de 8,9 g d'un mélange des deux racémates thréo et érythro de 2,3-dihydro 2-oxirannyl) 1,4-benzodioxin, 8,7 g de 3-(pipéridin-4-yl) 2H-indol 2-one, 100 ml de benzène anhydre, 2 ml de méthanol et 0,1 g d'hydroquinone, élimine le benzène et le méthanol sous vide, reprend le résidu par de l'acétate d'éthyle, puis élimine ce solvant, reprend à nouveau par 20 ml d'acétate d'éthyle, et maintient au réfrigérateur pendant trois jours, obtient 8,6 g de produit que l'on purifie par recristallisation dans 40 ml d'isopropanol et obtient 6 g de produit attendu. F = 115—120°C.

*Analyse*: $C_{23}H_{26}N_2O_4$

| | | | |
|---|---|---|---|
| Calculé : | C% 70,03 | H% 6,64 | N% 7,10 |
| Trouvé : | 69,8 | 7,0 | 6,9 |

Spectre RMN (Deutérochloroforme, 60 MHz)

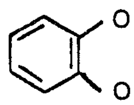 à 416 Hz

— autres aromatiques à 410—450 Hz
— $CH_2O$ et —CHO à 220—275 Hz
— hydrogènes mobiles à 530 et 207 Hz
— autres protons = 80 à 195 Hz

*Préparation du chlorhydrate*

On dissout les 6 g de produit obtenu ci-dessus dans 100 ml d'acétone et y ajoute 4,5 ml d'éther chlorhydrique 5N, élimine le solvant sous vide, reprend le produit brut obtenu par 100 ml d'éthanol à chaud et obtient 4,3 g de produit attendu. F = 160°C (point de fusion peu net).

*Analyse:* $C_{23}H_{27}ClN_2O_4$

| | | | | |
|---|---|---|---|---|
| Calculé : | C% 64,10 | H% 6,32 | N% 6,50 | Cl% 8,23 |
| Trouvé : | 63,8 | 6,7 | 6,3 | 8,4 |

La 3-(pipéridin-4-yl) 2H-indol 2-one utilisée au départ de la préparation du produit ci-dessus, a été préparée de la manière suivante:

Stade A

*3-/1-benzyl 4-pipéridylène/ 2H-indol 2-one*

On mélange sous agitation 20 g d'indol 2-one, 29,2 g de N-benzyl 4-pipéridone, 420 ml d'éthanol, fait barboter de l'ammoniac pendant une demi-heure, chauffe vers 80—90°C de façon à réduire le volume d'éthanol de moitié en 2 heures, refroidit la solution, ajoute 200 ml d'eau, filtre, lave le précipité par un mélange eau-méthanol (1-1), sèche et obtient 34 g de produit attendu dont on recristallise un échantillon dans le xylène. F. = 218°C.

Stade B

*3-(pipéridin-4-yl) 2H-indol 2-one*

On introduit dans une cellule à hydrogéner, 38,1 g de 3-(1-benzyl) 4-pipéridylène 2H-indol 2-one obtenue au stade A, 4 g de palladium sur charbon à 10%, 250 ml d'acide acétique et chauffe à 50°C. A la fin de la réaction, on filtre, amène à sec, reprend le résidu par 500 ml d'eau, traite par de l'hydroxyde de sodium, extrait la base par 6 fois 200 ml d'éther, après avoir saturé la phase aqueuse au chlorure de sodium. On sèche la phase éthérée sur sulfate de sodium, amène à sec, distille l'huile obtenue (Eb = 192—194°C), et obtient 11 g de produit attendu amorphe.

On a réalisé une deuxième préparation du produit dans les mêmes conditions et obtenu un produit brut que l'on a dissous dans du benzène à chaud et reprécipité au cyclohexane. F. = 156°C.

Exemple 2

*(dl) érythro 1,3-dihydro 3-/1-/2-(2,3-dihydro 1,4-benzodioxin-2-yl) 2-hydroxy éthyl/ pipéridin-4-yl/ 2H-indol 2-one et son chlorhydrate*

On porte au reflux sous agitation pendant 4 heures un mélange de 4,35 g de 3-(4-pipéridinyl) 2H-indol 2-one, 4,5 g de (dl) érythro 2,3 dihydro 2-oxirannyl 1,4-benzodioxine, 50 ml de benzène et 5 ml de méthanol, refroidit, élimine les solvants sous vide, obtient 8,1 g de produit que l'on chromatographie sur une colonne de silice en éluant par un mélange chlorure de méthylène-méthanol (95/5), obtient 5,1 g de produit attendu qui cristallise après une nuit au réfrigérateur. Point de fusion peu net vers 90—100°C.

*Spectre RMN* (Deutérochloroforme, 60 MHz)
— $CH_2O$ et $>CH—O$: 200 à 270 Hz
— $CH_2$ et H angulaire = 80 à 190 Hz

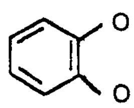 à ≃ 410 Hz

— autres aromatiques: de 410 à 440 Hz.

*Préparation du chlorhydrate*

On dissout les 5,1 g de produit obtenu ci-dessus dans 20 ml d'isopropanol, ajoute 3 ml d'une solution 5N d'éther chlorhydrique, concentre et reprend le résidu dans 100 ml d'éther. Le produit cristallise après 48 h. sous agitation. On essore, lave à l'éther et sèche.

*Analyse:* $C_{23}H_{27}ClN_2O_4$

| | | | | |
|---|---|---|---|---|
| Calculé : | C% 64,10 | H% 6,32 | Cl% 8.23 | N% 6,50 |
| Trouvé : | 64,0 | 6,5 | 8,0 | 6,2 |

Exemple 3

*(dl) thréo 1,3-dihydro 3-/1-/2-(2,3-dihydro 1,4-benzodioxin-2-yl) 2-hydroxy éthyl/ pipéridin-4-yl/ 2H-indol 2-one et son chlorhydrate*

On porte au reflux pendant 18 heures un mélange de 8,7 g de 3-(4-pipéridinyl) 2H-indol 2-one, 8,6 g de (dl) thréo 2,3-dihydro 2-oxirannyl 1,4-benzodioxine, 100 ml de benzène et 10 ml de méthanol, ajoute 10 ml d'éther chlorhydrique 4N, élimine les solvants par décantation et cristallise le produit dans 30 ml d'alcool isopropylique, obtient 17,2 g de produit attendu. F. ≃ 180°C.

*Préparation du chlorhydrate*

On dissout le produit obtenu ci-dessus dans 200 ml de méthanol chaud, filtre, amène à température ambiante, ajoute 400 ml d'éther chlorhydrique, essore et sèche le précipité et obtient 7,35 g du chlorhydrate attendu. F. ≃ 205°C.

*Analyse:* $C_{23}H_{27}ClN_2O_4$

| | | | | |
|---|---|---|---|---|
| Calculé : | C% 64,10 | H% 6,31 | Cl% 8,22 | N% 6,50 |
| Trouvé : | 63,7 | 6,6 | 8,1 | 6,2 |

*Spectre RMN* (Diméthylsulfoxyde) fréquence de base = 90 MHz
— hydrogènes mobiles: 946—810—540—546 Hz
— aromatiques: 615 à 660 Hz avec pic à 618 Hz
— $CH_2O$ et CH—O: 350 à 400 Hz
— $CH_2$—N: 250 à 320 Hz
— autres protons: 125 à 220 Hz.

Exemple 4

*(dl) érythro 1,3-dihydro 3-/1-/2-(2,3-dihydro 1,4-benzodioxin-2-yl) 2-hydroxy éthyl/ pipéridin-4-yl/1-méthyl 2H-indol 2-one et son chlorhydrate*

On porte au reflux sous agitation pendant 20 heures un mélange de 9,2 g de 1,3-dihydro 3-(pipéridin-4-yl) 1-méthyl 2H-indol 2-one, 8,6 g d'érythro 2-oxyrannyl 2,3-dihydro 1,4-benzodioxine, 100 ml de benzène anhydre et 10 ml de méthanol, élimine le benzène et le méthanol sous vide, et chromatographie sous pression le résidu en éluant au mélange chlorure de méthylène-méthanol (96:4). On obtient 11,4 g de produit attendu brut.

*Analyse:* $C_{24}H_{28}N_2O_4$

| | | | |
|---|---|---|---|
| Calculé : | C% 70,55 | H% 6,91 | N% 6,86 |
| Trouvé : | 70,8 | 6,9 | 6,8 |

*Spectre RMN* (Deutérochloroforme, 60 MHz)
— N—$CH_3$ à 190,5 Hz
— —CHO et $CH_2O$ de 210 à 270 Hz
— —CH—C=O à 203—209 Hz
— autres protons de 75 à 180 Hz.

*Préparation du chlorhydrate*

On dissout 11 g de produit obtenu ci-dessus dans 100 ml d'éther et y ajoute 6 ml d'une solution d'éther chlorhydrique 5N, essore le chlorhydrate précipité, recristallise dans 60 ml de méthanol et obtient 5,3 g de chlorhydrate attendu. F = 250°C.

*Analyse:* $C_{24}H_{29}ClN_2O_4$

| | | | | |
|---|---|---|---|---|
| Calculé : | C% 64,78 | H% 6,57 | Cl% 7,97 | N% 6,30 |
| Trouvé : | 64,6 | 6,4 | 7,8 | 6,3 |

La 1,3-dihydro 1-méthyl 3-(pipéridin-4-yl) 2H-indol 2-one utilisée au départ de la préparation ci-dessus, a été préparée de la manière suivante:

Stade A

*1,3-dihydro 1-méthyl 3-/1-benzyl pipéridin-4-ylidène/ 2H-indol 2-one*

On mélange 67 g de N-méthyl indol 2-one, 95 g de N-benzyl 4-pipéridone et 1000 ml d'éthanol, fait barboter de l'ammoniac pendant 2 heures à température ambiante et laisse une nuit au repos. On chauffe au reflux pendant 8 heures en maintenant un léger courant d'ammoniac. On élimine le solvant sous vide et reprend le résidu par 500 ml d'acide chlorhydrique 2N, filtre, lave à l'eau le chlorhydrate du produit attendu, reprend par 1 litre d'eau et 500 ml d'éther, ajoute 100 ml de l'hydroxyde de sodium concentré et maintient sous agitation pendant 5 heures. On décante la phase organique qu'on lave à l'eau; on sèche, amène à sec et obtient 91 g de produit attendu que l'on recristallise dans 250 ml d'alcool isopropylique. On obtient alors 59,7 g de produit attendu. F. = 101—102°C.

*Analyse* :

| | | | |
|---|---|---|---|
| Calculé : | C% 79,21 | H% 6,96 | N% 8,80 |
| Trouvé : | 78,9 | 7,0 | 8,7 |

Stade B

*1,3-dihydro 3-(pipéridin-4-yl)2H-indol 2-one*

On mélange 16 g de 1,3-dihydro 1-méthyl 3-/1-benzyl pipéridin-4-ylidène/ 2H-indol 2-one, 150 ml d'acide acétique, 2 g de palladium sur charbon à 10%, porte à 50°C et sature d'hydrogène. A la fin de la réaction, après 2 heutres, on filtre, amène à sec, reprend le résidu par l'eau et traite par l'hydroxyde de sodium concentré, extrait par 4 fois 100 ml de chlorure de méthylène, lave les phases

organiques avec peu d'eau, sèche, concentre à sec et obtient 9,5 g de produit attendu. F. ≃ 80°C.

*Analyse:*

| | | | |
|---|---|---|---|
| Calculé : | C% 73,01 | H: 7,88 | N% 10,16 |
| Trouvé : | 72,6 | 7,8 | 11,8 |

Example 5

*(dl)-érythro 1,3-dihydro 3-/1-/2-(2,3-dihydro 1,4-benzodioxin-2-yl) 2-hydroxyéthyl/ pipéridin-4-yl/ 5-méthoxy 2H-indol 2-one et son chlorhydrate*

On porte au reflux pendant 3 heures un mélange de 9,85 g de 1,3-dihydro 5-méthoxy 3-(pipéridin-4-yl) 2H-indol 2-one, 8,6 g de érythro 2-oxyrannyl 1,4-benzodioxine, 100 ml de benzène anhydre, 5 ml de méthanol, élimine le benzène et le méthanol sous vide, chromatographie sur silica en éluant au mélange chlorure de méthylène-méthanol (95-5), isole le produit, le cristallise dans l'éther et obtient 14,2 g de produit attendu. F. = 110°C (fusion pâteuse).

*Analyse:*

| | | | |
|---|---|---|---|
| Calculé : | C% 67,89 | H% 6,65 | N% 6,60 |
| Trouvé : | 67,1 | 6,6 | 6,5 |

*Spectre RMN* (Deutérochloroforme, 60 MHz)
— hydrogène du cycle azoté à 201—203 Hz
— $CH_2O$ et CHO de 220 à 270 Hz
— $OCH_3$ à 228 Hz
— aromatiques de 405 à 411 Hz

*Préparation du chlorhydrate*

On dissout le produit obtenu ci-dessus dans 100 ml de chlorure de méthylène et ajoute 10 ml d'acide chlorhydrique 4N, puis 200 ml d'éther anhydre, essore, lave à l'éther et sèche, et obtient 11,8 g de chlorhydrate attendu. F. = 150°C (fusion pâteuse).

La 1,3-dihydro 5-méthoxy 3-(pipéridin-4-yl) 2H-indol 2-one, utilisée au départ de la préparation du produit ci-dessus, a été préparée de la manière suivante:

Stade A

*1,3-dihydro 5-méthoxy 3-(1-benzyl pipéridin-4-ylidène) 2H-indol 2-one*

On mélange 10 g de 1,3-dihydro 5-méthoxy 2H-indol 2-one, 200 ml d'éthanol, 20 ml de N-benzyl 4-pipéridone, fait barboter de l'ammoniac pendant une heure, chauffe vers 90°C pendant 4 heures, laisse une nuit à température ambiante, dilue à l'eau, extrait au chlorure de méthylène, lave à l'eau, sèche, amène à sec et purifie par chromatographie sur silice en éluant par un mélange cyclohexane-6 chloroforme-3 triéthylamine-1 et on obtient 12,4 g de produit attendu. F = 152°C.

Stade B

*1,3-dihydro 5-méthoxy 3-(pipéridin-4-yl) 2H-indole 2-one*

On mélange en agitant 14 g de produit obtenu au Stade A ci-dessus, 140 ml d'acide acétique, chauffe vers 50°C, ajoute 1,4 g de palladium à 9,6% sur charbon et sature d'hydrogène. A la fin de la réaction, on filtre, amène à sec, reprend le résidu par l'eau, traite par de l'hydroxyde de sodium, extrait au chlorure de méthylène, lave à l'eau, sèche, amène à sec et obtient 8,5 g de produit attendu brut. Chromatographie sur silice, éluant: chloroforme, méthanol, triéthylamine (85:10:5): Rf = 0,15.

*Etude pharmacologique*

1) Détermination de la toxicité aiguë

La toxicité aiguë a été déterminée sur un lot de 10 souris pesant de 18 g à 22 g. Le produit a été administré en suspension dans la carboxyméthyl cellulose par voie intrapéritonéale.

Les animaux ont été gardés en observation pendant une semaine.

On a déterminé la dose létale (50 DL 50) et obtenu les résultats suivants:

| Produit de l'exemple | DL 50 mg/kg |
|---|---|
| 1 (chlorhydrate) | ≃ 150 |
| 2 (chlorhydrate) | ≃ 125 |
| 3 (chlorhydrate) | ≃ 125 |
| 4 (chlorhydrate) | ≃ 200 |

2) Détermination de l'activité hypotensive

L'activité hypotensive a été étudiée sur des rats mâles de souche Sprague Dawley S.P.F. pesant 300 g environ et anesthésiés au nembutal (50 mg/kg par voie intraveineuse).

Le produit testé a été administré par voie intraveineuse dans la veine jugulaire.

La pression artérielle carotidienne a été mesurée avant et après administration du produit testé.

La tableau ci-après indique les variations exprimées en pourcentage de la pression artérielle, après administration du produit testé, par rapport à la pression artérielle initiale ainsi que le temps pendant lequel se manifestent ces variations.

| Produit de l'exemple | Doses (mg/kg) | Variation % de la pression artérielle | Durée d'action (minutes) |
|---|---|---|---|
| 1 (chlorhydrate) | 1 | - 20<br>- 10 | 1<br>45 |
| 2 (chlorhydrate) | 1 | - 30<br>- 20 | 5<br>60 |
| 3 (chlorhydrate) | 1 | - 20<br>- 10 | 1<br>15 |
| 4 (chlorhydrate) | 1 | - 28<br>- 23<br>- 26<br>- 24 | 1<br>5<br>10<br>30 |

3) Détermination de l'activité antihypertensive

L'action antihypertensive a été étudiée sur des rats mâles spontanément hypertendus (souche Okamoto) âgés de 8 semaines. Le produit à tester a été administré par voie orale quotidiennement pendant 9 jours.

La pression artérielle a été mesurée à la queue du rat au moyen d'un manchon pneumatique relié à un transducteur électronique de pression.

La pression a été mesurée avant et après administration du produit.

Le tableau ci-après indique les variations exprimées en pourcentages de la pression artérielle, après administration du produit par rapport à la pression initiale.

| Produit de l'exemple | Dose quotidienne (mg /kg) | Variation % de la pression artérielle | | |
|---|---|---|---|---|
| | | 1er jour | | 10è jour |
| | | 1h. après la 1ère administration | 4h. après la 1ère administration | 24h. après la dernière administration |
| 1 (chlorhydrate) | 50 | - 29 | - 27 | - 8 |
| 2 (chlorhydrate) | 50 | - 23 | - 22 | - 9 |
| *4 (chlorhydrate) | 50 | - 23 | - 31 | -16 |

* Dans ce cas, le produit a été administré par voie orale quotidiennement pendant 3 jours.

**Revendications**

1. Les produits de formule générale I:

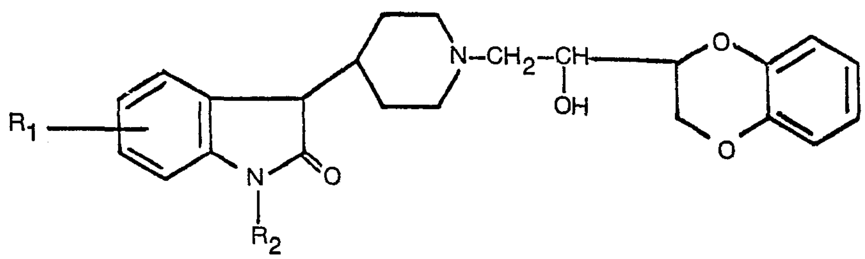

(I)

dans laquelle $R_1$ représente un atome d'hydrogène, un atome de chlore, de brome ou de fluor, un radical alcoxy contenant de 1 à 5 atomes de carbone, $R_2$ représente un atome d'hydrogène ou un radical alcoyle contenant de 1 à 5 atomes de carbone, sous leurs formes racémiques ou optiquement actives, ainsi que leurs sels d'addition avec les acides minéraux ou organiques.

2. Les produits tels que définis par la formule I de la revendication 1, caractérisés en ce que dans ladite formule I, $R_1$ représente un atome d'hydrogène, un atome de chlore ou un radical méthoxy, et $R_3$ représente un atome d'hydrogène ou un radical méthyle, sous leurs formes racémiques ou optiquement actives, ainsi que leurs sels d'addition avec les acides minéraux ou organiques.

3. Le produit de formule I selon la revendication 1 dénommé l'érythro 1,3-dihydro 3-/1-/2-(2,3-dihydro 1,4-benzodioxin-2-yl) 2-hydroxy éthyl/ pipéridin-4-yl/2H-indol 2-one et son chlorhydrate.

4. Le produit de formule I selon la revendication 1 dénommé thréo 1,3-dihydro 3-/1-/2-(2,3-dihydro 1,4-benzodioxin-2-yl) 2-hydroxy éthyl/ pipéridin-4-yl/2H-indol 2-one et son chlorhydrate.

5. Le produit de formule I selon la revendication 1 dénommé l'érythro 1,3-dihydro 3-/1-/2-(2,3-dihydro 1,4-benzodioxin-2-yl) 2-hydroxy éthyl/ pipéridin-4-yl 1-méthyl 2H-indol 2-one et son chlorhydrate.

6. Le produit de formule I selon la revendication 1 dénommé l'érythro 1,3-dihydro 3-/1-/2-(2,3-dihydro 1,4-benzodioxin-2-yl) 2-hydroxy éthyl/ pipéridin-4-yl 5-méthoxy 2H-indol 2-one et son chlorhydrate, sous leurs formes racémiques ou optiquement actives.

7. Procédé de préparation des produits tels que définis par la formule I de la revendication 1, sous leurs formes racémiques et optiquement actives, ainsi que de leurs sels d'addition avec les acides minéraux et organiques, caractérisé en ce que l'on fait réagir un produit de formule II

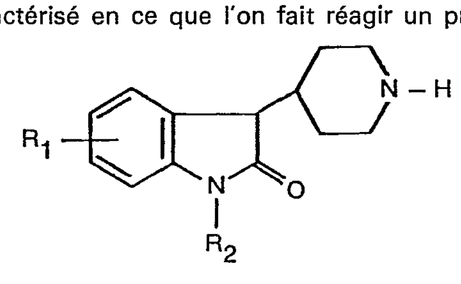

(II)

dans laquelle $R_1$ et $R_2$ ont la signification donnée à la revendication 1, avec un produit de formule III:

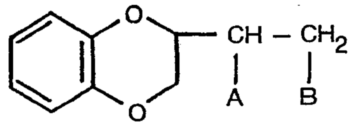

(III)

dans laquelle A et B représentent ensemble un atome d'oxygène ou bien A représente un radical hydroxy et B représente un atome de chlore ou de brome, pour obtenir le produit de formule I que, si désiré, on soumet à l'action d'un acide minéral ou organique pour en former le sel.

8. Procédé de préparation selon la revendication 7, caractérisé en ce que les produits de formule II sont préparés par réaction d'un produit de formule (A):

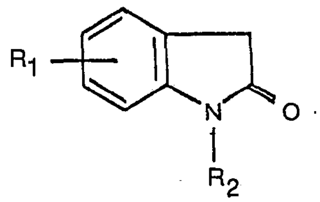

(A)

dans laquelle $R_1$ et $R_2$ ont la signification indiquée à la revendication 1, avec le produit de formule (B):

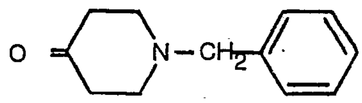

(B)

et traitement du produit de formule IV obtenu:

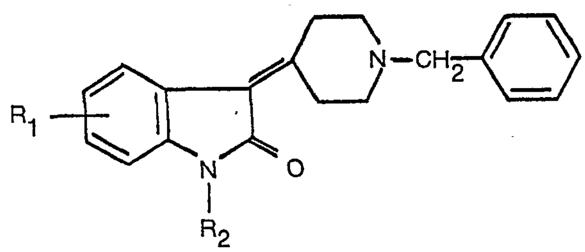

(IV)

par un agent de réduction et de débenzylation.

9. A titre de médicaments, les produits tels que définis par la formule I de la revendication 1, sous leurs formes racémiques et optiquement actives, ainsi que leurs sels d'addition avec les acides minéraux et organiques pharmaceutiquement acceptables.

10. Médicaments selon la revendication 9, caractérisés en ce que, dans la formule I, $R_1$ et $R_2$ ont la signification indiquée à la revendication 2.

11. A titre de médicaments, les produits tels que définis à la revendication 3, 4, 5 ou 6.

12. Les compositions pharmaceutiques contenant, à titre de principe actif, l'un au moins des médicaments, selon l'une quelconque des revendications 9 à 11.

**Claims**

1. The products of general formula I:

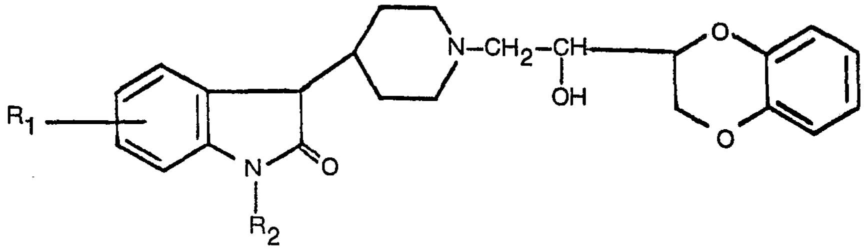

(I)

in which $R_1$ represents a hydrogen atom, a chlorine, bromine or fluorine atom or an alkoxy radical containing from 1 to 5 carbon atoms and $R_2$ represents a hydrogen atom or an alkyl radical containing

from 1 to 5 carbon atoms, in their racemic or optically-active forms, as well as their addition salts with mineral or organic acids.

2. The products as defined by the formula I of Claim 1, characterized in that, in the said formula I, $R_1$ represents a hydrogen atom, a chlorine atom or a methoxy radical and $R_2$ represents a hydrogen atom or a methyl radical, in their racemic or optically-active forms, as well as their addition salts with mineral or organic acids.

3, The product of formula I according to Claim 1, named erythro 1,3-dihydro 3-[1-[2-(2,3-dihydro 1,4-benzodioxin-2-yl) 2-hydroxy ethyl] piperidin-4-yl] 2H-indol 2-one and its hydrochloride.

4. The product of formula I according to Claim 1, named threo 1,3-dihydro 3-[1-[2-(2,3-dihydro 1,4-benzodioxin-2-yl) 2-hydroxy ethyl] piperidin-4-yl] 2H-indol 2-one and its hydrochloride.

5. The product of formula I according to Claim 1, named erythro 1,3-dihydro 3-[1-[2-(2,3-dihydro 1,4-benzodioxin-2-yl) 2-hydroxy ethyl] piperidin-4-yl] 1-methyl 2H-indol 2-one and its hydrochloride.

6. The product of formula I according to Claim 1, named erythro 1,3-dihydro 3-[1-[2-(2,3-dihydro 1,4-benzodioxin-2-yl) 2-hydroxy ethyl] piperidin-4-yl] 5-methoxy 2H-indol 2-one and its hydrochloride, in their racemic or optically-active forms.

7. Process for preparing the products as defined by the formula I of Claim 1, in their racemic and and optically-active forms, as well as their addition salts with mineral and organic acids, characterized in that a product of formula II:

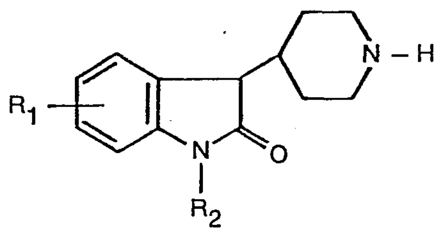

(II)

in which $R_1$ and $R_2$ have the meaning given in Claim 1, is reacted with a product of formula III:

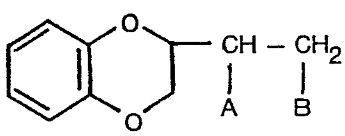

(III)

in which A and B together represent an oxygen atom or A represents a hydroxy radical and B represents a chlorine or bromine atom, to obtain the product of formula I which, if desired, is subjected to the action of a mineral or organic acid to form the salt thereof.

8. Preparation process according to Claim 7, characterized in that the products of formula II are prepared by the reaction of a product of formula A:

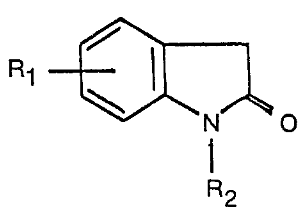

(A)

in which $R_1$ and $R_2$ have the meaning indicated in Claim 1, with the product of formula B:

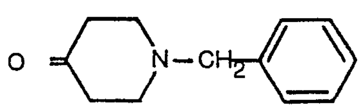

(B)

and treatment of the product of formula IV obtained:

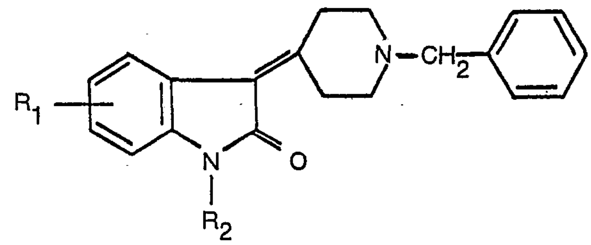

(IV)

with a reducing and debenzylating agent.

9. As medicaments the products as defined by the formula I of Claim 1, in their racemic and optically-active forms, as well as their addition salts with pharmaceutically-acceptable mineral and organic acids.

10. Medicaments according to Claim 9, characterized in that, in the formula I, $R_1$ and $R_2$ have the meaning indicated in Claim 2.

11. As medicaments the products as defined in Claim 3, 4, 5 or 6.

12. The pharmaceutical compositions containing, as active principle, one at least of the medicaments according to any one of Claims 9 to 11.

**Patentansprüche**

1. Produkte der allgemeinen Formel I

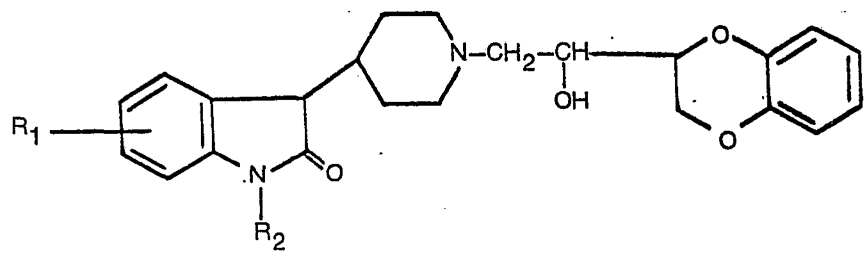

(I)

worin $R_1$ ein Wasserstoffatom, ein Chlor-, Brom- oder Fluor- atom oder einen Alkoxyrest mit 1 bis 5 Kohlenstoffatomen bedeutet, und $R_2$ ein Wasserstoffatom oder einen Alkylrest mit 1 bis 5 Kohlenstoffatomen darstellt, in ihren racemischen oder optisch aktiven Formen, sowie deren Additionssalze mit Mineral- oder organischen Säuren.

2. Produkte der Formel I gemäß Anspruch 1, dadurch gekennzeichnet, daß in der Formel I $R_1$ ein Wasserstoffatom, ein Chloratom oder einen Methoxyrest bedeutet, und $R_2$ ein Wasserstoffatom oder einen Methylrest bedeutet, in ihren racemischen oder optisch aktiven Formen, sowie deren Additionssalze mit Mineral- oder organischen Säuren.

3. Produkt der Formel I gemäß Anspruch 1 mit der Bezeichnung Erythro-1,3-dihydro-3-[1-[2-(2,3-dihydro-1,4-benzodioxin-2-yl)-1-hydroxyäthyl]-piperidin-4-yl]-2H-indol-2-on und dessen Hydrochlorid.

4. Produkt der Formel I gemäß Anspruch 1 mit der Bezeichnung Threo-1,3-dihydro-3-[1-[2-(2,3-dihydro-1,4-benzodioxin-2-yl)-2-hydroxyäthyl]-piperidin-4-yl]-2H-indol-2-on und dessen Hydrochlorid.

5. Produkt der Formel I gemäß Anspruch 1 mit der Bezeichnung Erythro-1,3-dihydro-3-[1-[2-(2,3-dihydro-1,4-benzodioxin-2-yl)-2-hydroxyäthyl]-piperidin-4-yl]-1-methyl-2H-indol-2-on und dessen Hydrochlorid.

6. Produkt der Formel I gemäß Anspruch 1 mit der Bezeichnung Erythro-1,3-dihydro-3-[1-[2-(2,3-dihydro-1,4-benzodioxin-2-yl)-2-hydroxyäthyl]-piperidin-4-yl]-5-methoxy-2H-indol-2-on und dessen Hydrochlorid in ihren racemischen oder optisch alktiven Formen.

7. Verfahren zur Herstellung der Produkte der Formel I gemäß Anspruch 1 in ihren racemischen und optisch aktiven Formen, sowie von deren Additionssalzen mit Mineral- und organischen Säuren, dadurch gekennzeichnet, daß man ein Produkt der Formel II

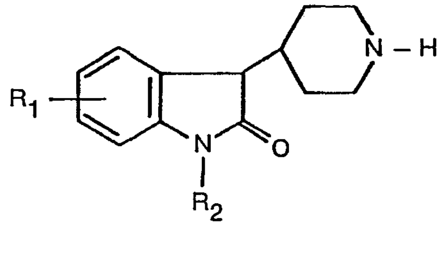

(II)

worin $R_1$ und $R_2$ die in Anspruch 1 angegebene Bedeutung besitzen, mit einem Produkt der Formel III

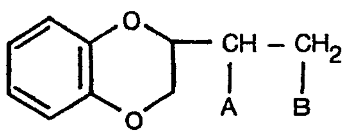

(III)

worin A und B zusammen ein Sauerstoffatom darstellen oder A einen Hydroxyrest bedeutet und B ein Chlor- oder Bromatom bedeutet, umsetzt, um das Produkt der Formel I zu erhalten, das man gewünschtenfalls der Einwirkung einer Mineral- oder organischen Säure unterzieht, um hieraus das Salz zu bilden.

8. Verfahren gemäß Anspruch 7, dadurch gekennzeichnet, daß die Produkte der Formel II hergestellt werden durch Umsetzung eines Produkts der Formel A

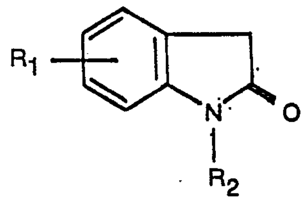

(A)

worin $R_1$ und $R_2$ die in Anspruch 1 angegebene Bedeutung besitzen, mit dem Produkt der Formel B

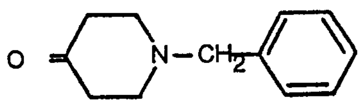

(B)

und Behandlung des erhaltenen Produkts der Formel IV

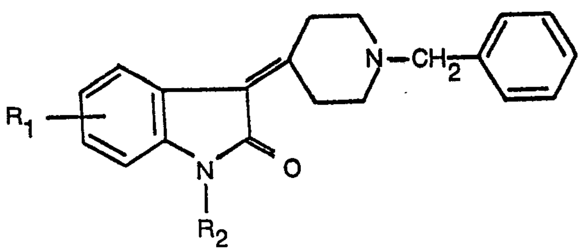

(IV)

mit einem Reduktions- und Debenzylierungsmittel.

9. Als Arzneimittel die Produkte der Formel I gemäß Anspruch 1, in ihren racemischen und optisch aktiven Formen, sowie deren Additionssalze mit pharmazeutisch verträglichen Mineral- und organischen Säuren.

10. Arzneimittel gemäß Anspruch 9, dadurch gekennzeichnet, daß in der Formel I $R_1$ und $R_2$ die in Anspruch 2 angegebene Bedeutung besitzen.

11. Als Arzneimittel die Produkte gemäß Anspruch 3, 4, 5 oder 6.

12. Pharmazeutische Zusammensetzungen, enthaltend als Wirkstoff zumindest eines der Arzneimittel gemäß einem der Ansprüche 9 bis 11.